(19) Europäisches Patentamt
European Patent Office
Office européen des brevets

(11) Publication number: **0 031 051**
**A1**

(12) # EUROPEAN PATENT APPLICATION

(21) Application number: 80107452.7

(51) Int. Cl.³: **C 12 Q 1/58**

(22) Date of filing: 28.11.80

(30) Priority: 10.12.79 US 101772

(43) Date of publication of application: 01.07.81 Bulletin 81/26

(84) Designated Contracting States: CH DE FR GB IT LI SE

(71) Applicant: MILES LABORATORIES INC.
1127 Myrtle Street
Elkhart Indiana 46514(US)

(72) Inventor: Bergonzi, Carlo
Via V. Veneto, 8
I-20060 Mastate (MI)(IT)

(72) Inventor: Berti, Giovanni
Via Provincial, 34
I-22040 Malgrate (CO)(IT)

(72) Inventor: Imbasciati, Alberto
Via Lorenzina, 4
I-22040 Malgrate (CO)(IT)

(74) Representative: Senftl, Hannes, Dr. et al,
c/o Bayer AG Zentralbereich Patente Marken und Lizenzen
D-5090 Leverkusen 1, Bayerwerk(DE)

(54) **Urea assay and process and test-kit for determination of urea.**

(57) Test means, such as a composition, a test device, method of making a test device and process for detection of urea are disclosed. More particularly, suitable test means are of the type which comprise urease, and a reagent composition comprising acetylacetone and a $C_1$-$C_4$ alkyl aldehyde or the reaction product 3,5-diacetyl-2,6-heptanedione of acetylacetone and formaldehyde. The compositions are advantageously incorporated with a carrier to provide a test device and can be combined in a test kit for determining urea which, in packaged combination, comprises a container of urease, and a container of a reagent according to the invention.

EP 0 031 051 A1

Croydon Printing Company Ltd.

TITLE MODIFIED
see front page

# UREA ASSAY
# AND PROCESS FOR DETERMINATION OF UREA

## *FIELD OF THE INVENTION*

The present invention relates generally to the field of diagnostic compositions, and more particularly, to diagnostic tests useful in the qualitative and quantitative determination of urea in body fluids, especially blood.

## *BACKGROUND OF THE INVENTION*

Urea is the major end product of protein metabolism in man and in other mammals. This product is formed in the liver, passes into the blood and is excreted through the kidney into the urine, where it constitutes the major fraction of the organic substances. Elevations of blood urea, referred to as azotemia, almost invariably indicate impairment of renal function. The test for determination of blood urea (usually expressed as urea nitrogen in the United States) is one of the most performed in routine clinical chemistry. Urea concentration in urine is also sometimes measured for determining the urea clearance, which is a measure of the glomerular filtration rate.

Methods used in routine clinical chemistry for determining urea in biological fluids can be classified as direct (condensation of urea with suitable reagents able to form a measurable chromogen) and indirect (determination of ammonia as a product of urease action on urea).

Among direct methods, the one with diacetylmonoxime, described in R. J. Henry, *et al* Clinical Chemistry - Principles and Technics, Harper & Row 2nd Edition (1974), has been widely applied in routine clinical chemistry, both with manual and automated procedures. This method suffers many disadvantages, such as:

(1). relative non-specificity, since it gives positive reactions with citrulline, allantoin, and other body fluids components;

(2). a standard curve (or at least a two-point calibration) must be prepared every day;

(3). the color fades rapidly and is photosensitive;

(4). the reaction has to be carried out at a relatively high temperature; and

(5). the unpleasant odor and irritant fumes of the reagents make it advisable to work in a fume hood.

For these reasons, the enzymatic (indirect) methods are generally preferred to the diacetylmonoxime method.

Indirect methods are based on enzymatic conversion of urea to ammonia by urease (urea amidohydrolase) according to the reaction:

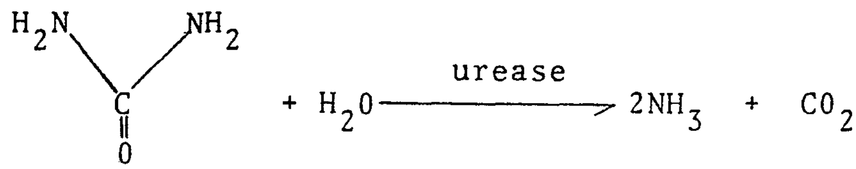


and subsequent determination of the liberated ammonia with suitable reagent systems [R. Richterich, "Clinical Chemistry-Theory and Practice", translated from the 2nd German Edition, S. Karger, Basel (1969) and H.U. Bergmeyer, "Methods of Enzymatic Analysis", 2nd English Edition, Vol. 4, Verlag Chemie - Academic Press, New York (1974)]. The chief characteristic of enzymatic methods is the specificity since only urea is hydrolyzed by urease.

Among the methods for determination of the enzymatically liberated ammonia, which is proportional to the concentration of urea in the sample, the one that has achieved the largest popularity in laboratory practice is based on the Berthelot reaction in which ammonium ions react in alkaline medium with phenol and hypochlorite to give the blue dye indophenol. The determination of urea according

to this method requires two steps: in the first step the sample is incubated at 37°C (usually from 10 to 20 minutes) with urease at the pH most suitable for the enzymatic reaction (i.e. around 6.5); in the second step alkaline solutions of phenol and hypochlorite are added and incubation carried out for developing color, usually at 37° C, from 10 to 30 minutes.

Another two-step method is described by Bergmeyer, *supra,* which allows determination of ammonium ions derived from the enzymatic reaction with urease by the enzyme glutamate dehydrogenase (GlDH) according to the reaction:

$$\text{2-oxoglutarate} + \text{NADH} + NH_4^+ \xrightarrow{\text{GlDH}} \text{L-glutamate} + NAD^+ + H_2O$$

and measurement of the decrease in the NADH absorbance at 340 (or 334, or 366) nanometers (nm). Various changes have been made in this method in order to perform it in only one step, with only one reagent preparation and making use of the kinetic type of analysis. In spite of the improvements this method remains of limited use in routine clinical chemistry owing to the high cost of the reagents and lack of applicability to automated continous-flow analyzers.

From another field of investigation, the "Hantzsch's dihydropyridine synthesis" forms a yellow product known as 3,5-diacetyl-1,4-dihydrolutidine (DDL) and is illustrated as follows:

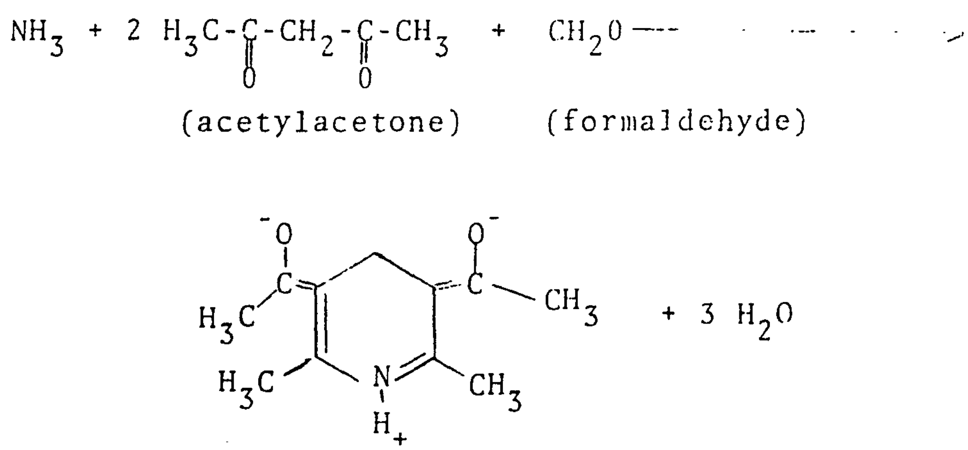


It has been applied to the analytical determination of formaldehyde (ammonia and acetylacetone as reagents) by Nash in Biochem J., 55: 416-421 (1953). Applications to clinical chemistry were also published, in which the determination of formaldehyde by this reaction is the final stage of complex reactions to determine cholesterol [P. Roeschlau, *et al*, Z. Klin. Chem. Klin. Biochem., 12 (9): 403, 407 (1974)], uric acid [N. Kageyama, Clin. Chim. Acta, 31: 421-426 (1971)] and triglycerides [G. Kessler, *et al*, Technicon Symposium 2nd, N.Y., London 1965, 341-344 (1966)]. A fluorimetric method for determination of ammonia using acetylacetone and formaldehyde was described in Belman, S., Anal. Chim. Acta, 29: 120-126 (1963).

## *OBJECTS OF THE INVENTION*

It is an object of the present invention to provide an improved test for detection of urea.

It is another object of the invention to provide a urea test comprising urease and a reagent composition comprising acetylacetone and a $C_1$-$C_4$ alkyl aldehyde.

It is yet another object of the invention to provide test means for the determination of urea which comprises urease and 3,5-diacetyl-2,6-heptanedione.

Other objects of the invention will be evident from the following description and claims.

## *SUMMARY OF THE INVENTION*

Present laboratory methods for assaying urea nitrogen in biological fluids suffer many disadvantages, already outlined. In contrast, the method according to the present invention is the only one that joins together the following advantageous characteristics: applicability to automated analyzers, including continous-flow analyzers (particularly with the enzyme urease in immobilized form); acceptable cost of reagents; determination in only one step; kinetic analysis both as a one-point and as a two-point (or multi-point) determination; low number of reagent preparations; and specificity.

The presence of all these characteristics contemporaneously in this method make it remarkably valuable in laboratory practice, both with manual methods and with automated or semi-automated instruments.



BAD ORIGINAL

In accordance with the present invention there are provided test means, such as a composition, a test device, a test kit, a method of making a test device and a process for detection of urea. More particularly, suitable test means are of the type which comprises urease and a reagent composition comprising acetylacetone and a $C_1$-$C_4$ alkyl aldehyde or the reaction product (3,5-diacetyl-2,6-heptanedione) of the acetylacetone and formaldehyde.

The composition can be incorporated with a carrier, such as a matrix or tablet, to provide a test device. The composition can also be combined in a test kit for determining urea which kit comprises, in packaged combination, a container of urease, a container of a reagent composition according to the invention and, optionally, a container of buffer.

## *DESCRIPTION OF THE PREFERRED EMBODIMENTS*

Although specific terms are used for clarity, these terms refer only to the particular embodiment selected for illustration, and are not intended to limit the scope of the invention.

In one embodiment, test means for the determination of urea in a sample comprise urease and an indicator reagent composition comprising acetylacetone and a $C_1$-$C_4$ alkyl aldehyde. The alkyl aldehyde can preferably be formaldehyde or acetaldehyde. In another embodiment the reaction product

resulting from reacting acetylacetone and formaldehyde, which reaction product is 3,5-diacetyl-2,6-heptanedione, can likewise be used as the indicator reagent composition for the determination. The synthesis of 3,5-diacetyl-2,6-heptanedione from acetylacetone and formaldehyde was described in B.D. Wilson, *et al.*, Belgian Pat. 618,140 and B.D. Wilson, J. Org. Chem., 28, 314-320 (1963), however this compound was not synthetized for analytical purposes.

The composition can be used in solution or in a variety of conventional dry formats. Whether as a solution or in a dry format, the composition according to the invention is used to detect urea by contacting it with a body fluid specimen such as urine, blood, plasma, or serum. When urea is present in the specimen to be tested a color change is effected thereby. Since a characteristic color reaction takes place depending on the concentration of the urea to be detected, quantitative determination of the urea is possible.

Also provided is a test device incorporating the composition of the invention and a single dip method of making such a reagent test device which comprises contacting a carrier, such as a tablet or matrix, with a solution of the composition according to the invention. When this contact is by impregnation with a solution of the composition according to the invention the carrier so contacted is then dried. The solvents used in preparing impregnation solution can include water, physiological solutions, organic solvents or mixtures thereof.



BAD ORIGINAL

The test device is advantageously used by momentarily dipping it in a test sample, or by otherwise introducing a test sample into the carrier, whereby a detectable color change results when urea is present. The test device may be used in the same way when samples of plasma, serum, or other body fluids are tested. The test device of the invention may be mounted on an elongated support member, if desired.

The term carrier refers to matrices which are insoluble in and maintain there structural integrity when exposed to physiological or other liquid. Suitable matrices which may be used include paper, cellulose, wood fabrics, gelatin, various organic polymer, such as polypropylene, and other organic materials well known as film formers to those skilled in the art. For convenience, the matrix can be associated with an insoluble support member such as can be made of polystyrene. The term carrier also refers to conventional tableting materials which can be pressed or molded into a convenient physical form.

Further in accordance with the invention there is provided a test kit for determining urea in a sample which comprises, in packaged combination, a container of urease, a container of an indicator reagent composition comprising acetylacetone and $C_1$-$C_4$ alkyl aldehyde and, optionally, a container of buffer. Preferably the alkyl aldehyde is

formaldehyde or acetaldehyde. In another embodiment the test kit for determination of urea comprises, in packaged combination, a container of urease, a container of 3,5-diacetyl-2,6-heptanedione and, optionally, a container of buffer. In either of these embodiments the test kit can further comprises a container of urea standard for comparison with the sample upon reaction with the composition or test device. Additionally, the test kit can be such that at least one of the containers is a cuvette, preferably one which is optically clear and can be used for spectrophotometric reading of the reaction resultant from contacting the composition with a sample.

For solutions containing the composition of the invention certain ranges are preferred. When the assay is performed with the composition in solution, the concentration ranges in the final aqueous solution of the assay are as follows. Urease can be used in concentrations giving an activity of from about 5 to about 60 U/ml [U is the unit of enzyme activity defined as the amount of activity that transforms 1 μmol of urea per minute at 25°C and pH = 8.0, according to the assay method of Schlegel and Kaltwasser in H.U. Bergmeyer, Methods of Enzymatic Analysis, 2nd English Edition, vol. 2, Verlag Chemie-Academic Press, New York (1974)] depending on the sample volume/reagent volume ratio; when this ratio is low (about 1/125), the preferred urease



BAD ORIGINAL

concentration is from 18 to 30 U/ml; when this ratio is high (about 1/20), the preferred urease concentration is from 30 to 50 U/ml.

The indicator reagent can be acetylacetone and a $C_1$-$C_4$ alkyl aldehyde or, likewise, it can be 3,5-diacetyl-2,6-heptanedione (DHD); in both cases the concentration is preferably as high as possible within the limits of solubility; if acetylacetone and a $C_1$-$C_4$ alkyl aldehyde are used as indicator reagent, the ratio between acetylacetone and the aldehyde is preferably stoichiometric (2 mol acetylacetone/1 mol aldehyde). A buffer substance can be included with the test means. The buffer substance can be used in concentrations of from about 0.01 to about 1.0 mol/l (mol/liter), preferably from 0.15 to 0.25 mol/l, and is preferably made with phosphate salts. Disodium edetate, in concentrations of from about 0.002 to about 0.008 mol/l, is preferably added. The pH of the solution can be from about 5.0 to about 9.0, preferably from 6.5 to 7.5.

The assay can be also performed with the composition of the invention in dry format. When the carrier is contacted with the composition by impregnating it with a solution of the composition, the concentration ranges in the aqueous solution for the impregnation are as follows. The activity (U/ml) of urease is preferably as high as possible within the limits of solubility. The indicator reagent, the buffer



BAD ORIGINAL

substance and disodium edetate are used with the same concentration ranges described for the assay performed with the composition in solution. A low boiling solvent soluble in water, like methanol, is preferably added in concentrations of from about 20 to about 30 ml/dl.

The manner of performing the determination of urea with the composition of the invention in solution can vary depending on whether the determination is manual or automated and which type of instrumentation is available. The basic distinction is between (A) equilibrium methods of analysis and (B) kinetic methods of analysis.

In the equilibrium method the reaction is carried out at about 37°C and after a suitable time the absorbance of the solution is read with a spectrophotometer at the suitable wavelength. Since the time for the reaction to reach the equilibrium (end-point) is very long, the assay is preferably performed in concentrated solution, in order to have a sufficient reaction rate and the reading is done at a point of quasi-equilibrium. When the reaction approaches the end and the rate is sufficiently low, the solution is diluted at room temperature with the amount of water necessary for the absorbance to be into the instrument scale and for the reaction rate to stop completely. Then, the absorbance of the solution is read at room temperature against a reagent blank. The calculation is done in the usual way by means of a standard.

In the one-point kinetic method the reaction is carried out at about 37°C and after a predetermined fixed time (before equilibrium) the absorbance of the solution is read with a spectrophotometer at the same temperature at the suitable wavelength against a reagent blank. If the time at which the absorbance is read is perfectly reproducible, as in automatic analyzers, the absorbance is linear with the analyte concentration. This linearity is valid at any time during the reaction; therefore, the reading can be done at any time, according to the required sensitivity. The calculation is done by means of a standard. With this method it is convenient, particularly if serum or plasma is used as sample, to use a sample volume/reagent volume ratio not too high, in order to avoid intereferences from the sample. Therefore, a long reading time can be necessary to reach the required sensitivity. In this case it is convenient to use automatic analyzers that read the absorbance of the samples one after the other without waiting for the incubation time between sample and sample (for example, continous-flow analyzers).

In the two-point (or multi-point) method the reaction is carried out preferably at about 37°C and the absorbance change of the solution over a predetermined fixed time interval (before the equilibrium) is measured with a spectrophotometer at the same temperature at the suitable wavelength against a reagent blank. If the time interval over

which the absorbance change is measured is perfectly reproducible, as in automatic analyzers, the absorbance change is linear with the analyte concentration. This linearity is valid over any time interval during the reaction; therefore the measurement can be done over any time interval, according to the required sensitivity. The calculation is done by means of a standard. With this method interferences from substances present in the sample, absorbing at the reading wavelength, are eliminated even if a high sample volume/reagent volume ratio is used. Therefore the required sensitivity can be reached with very low measurement times, and any type of automatic analyzers functioning with the two-point (or multi-point) method can be used.

In the above described methods of analysis, the absorbance of a solution is measured and this accords with an assay performed with the composition of the invention in solution. When the assay is performed with this composition in dry format and the color develops on an opaque surface, the above described methods can still be used, provided that a suitable instrument measures the reflectance of this surface instead of the absorbance of the solution. Alternatively a qualitative or a semi-quantitative reading can be performed visually, preferably with the aid of a suitable color scale.

The following examples illustrate preferred embodiments of the invention.

## Example 1

### Assay of Urea Nitrogen in Body Fluids with 3,5-Diacetyl-2,6-Heptanedione (DHD)

The DHD used in the assay was first synthetized as follows: 136 ml acetylacetone and 52 ml formaldehyde 35% (both analytical grade reagents) were dissolved in distilled water, made up to 1.0 liter and left stand at room temperature for 6 days (yield of DHD: about 92%. The contents of DHD was determined by UV analysis with the aid of a standard solution of DHD). The little amount of unreacted formaldehyde was removed by a slight vacuum. The resulting solution proved suitable for the use as an analytical reagent without further purification.

Reagent preparations used in the urea assay were as follows:

Reagent 1: Buffer $KH_2PO_4/K_2HPO_4$ 0.28 M in distilled water, pH 7, containing 0.2 g/dl disodium edetate dihydrate (analytical grade reagents)

Reagent 2: Urease, freeze-dried powder, 50 U/mg (from Miles Laboratories, Inc., code 36-615)

Reagent 3: The DHD solution above prepared

A working solution was prepared as follows:

Reagent 1 + 2: 150 mg reagent 2 were dissolved in 100 ml reagent 1 (stability: 3 months at 2-8°C)

The determination was performed according to the following methods:

(A) Kinetic two-point method

The volumes, in microliters (µl), used in the assay (volumes could be proportionally modified, when required) were as follows:

| | Blank | Sample | Standard |
|---|---|---|---|
| Sample (serum, plasma or diluted 1:100 urine) | | 50 µl | |
| Reagent 1 + 2 | 600 µl | 600 µl | 600 µl |
| Reagent 3 | 400 µl | 400 µl | 400 µl |
| Standard 20 mg/dl urea nitrogen in distilled water | | | 50 µl |
| Distilled water | 50 µl | | |

Only one blank and one standard were carried out for each series of tests.

The reaction was carried out at 37°C and the absorbance read at 410 nm with an automatic analyzer (1st reading after 30 seconds, 2nd reading after 120 seconds from the start). The calculation for serum or plasma samples was done by the following formula:

$$\text{Urea Nitrogen (mg/dl)} = \frac{\Delta E_{sample} - \Delta E_{blank}}{\Delta E_{standard} - \Delta E_{blank}} \times 20$$

where $\Delta E$ was the increase of absorbance in the given time interval. An aqueous standard was used since plasma proteins do not significantly interfere. For urine samples the results were multiplied by 100.

The method was linear up to at least 120 mg/dl urea nitrogen in the sample. Recovery tests carried out adding known amounts of urea to a pool of human plasmas gave an average percent recovery of 99.0 in the range of linearity. Assays of human sera or plasmas carried out in comparison with the urease/Berthelot method, gave a correlation coefficient r = 0.997 in the range of linearity.

(B) Kinetic one-point method

The volumes used in the assay (volumes could be proportionally modified, when required) were as follows:

| | Blank | Sample | Standard |
|---|---|---|---|
| Sample (serum, plasma or diluted 1:100 urine) | | 20 µl | |
| Reagent 1 + 2 | 1.5 ml | 1.5 ml | 1.5 ml |
| Reagent 3 | 10. ml | 1.0 ml | 1.0 ml |
| Standard 20 mg/dl urea nitrogen in distilled water | | | 20 µl |
| Distilled water | 20 µl | | |

Only one blank and one standard were carried out for each series of tests.

The reaction was carried out at 37°C and the absorbance at 410 nm read with an automatic analyzer after 10 minutes from the start. The calculation for serum or plasma samples was done by the following formula:

$$\text{Urea nitrogen (mg/dl)} = \frac{E_{sample} - E_{blank}}{E_{standard} - E_{blank}} \times 20$$

where E was the absorbance at the given reading time. An aqueous standard was used since plasma proteins do not significantly interefere. For urine samples the results were multiplied by 100.

The method was linear up to at least 120 mg/dl urea nitrogen in the sample. Recovery tests carried out adding known amounts of urea to a pool of human plasmas gave an average percent recovery of 99.3 in the range of linearity. Assays of human sera or plasmas carried out in comparison with the urease/Berthelot method, gave a correlation coefficient r = 0.995 in the range of linearity.

(C) Equilibrium method

The volumes used in the assay were the same as in the previous method. The reaction was carried out at 37°C and, after 1 1/2 hours, the solution was removed from the incubator and diluted to 10 ml with water and the absorbance read at 410 nm with a spectrophotometer. The calculation was the same as in the previous method.

The method was linear up to at least 120 mg/dl urea nitrogen in the sample. Recovery tests carried out adding known amounts of urea to a pool of human plasmas gave an average percent recovery of 98.4 in the range of linearity. Assays of human sera or plasmas carried out in comparison with urease/Berthelot method gave a correlation coefficient r = 0.986 in the range of linearity.

Thus, DHD has been shown to give reliable results in determinating urea nitrogen in both a one-step and a two-step kinetic assay as well as in the equilibrium method. Results correlated very well with those obtained using the standard Berthelot method and were free of the disadvantages in prior urea assay methods.

## Example II

Assay of Urea Nitrogen in Body Fluids with Acetylacetone and Formaldehyde

Reagent preparations used in the assay were as follows:

Reagent 1: the same as in Example 1

Reagent 2: the same as in Example 1

Reagent 3: Acetylacetone (analytical grade)

Reagent 4: Formaldehyde 35% (analytical grade)

Working solutions were prepared as follows:

Reagent 1 + 2: the same as in Example 1

Reagent 3 + 4: 13.6 ml of reagent 3 and 5.2 ml of reagent 4 were mixed and made up to 100 ml with distilled water.

The determination was performed by the same methods described in Example 1, using the same assay procedures and obtaining similar performances. Linearities were the same, percent recoveries and correlation coefficients were very close to those of Example 1.

## Example III

### Test Device Assay of Urea Nitrogen in Body Fluids with DHD

DHD was first synthetized as follows:

100 ml acetylacetone and 39 ml formaldehyde 35% were dissolved in 50 ml ethanol and let stand at room temperature for 3 days. The solvent was removed under vacuum and 63 g DHD were recovered as an oil by distillation (b.p. 120-125°C at 0.05 mm Hg). The elemental analysis calculated for $C_{11}H_{16}O_4$ was: C, 62,26; H, 7.55 and that found was: C, 61.21; H, 7.70.

Sheets of Eaton-Dikeman No. 204 filter paper were impregnated with the following solution:

| | |
|---|---|
| $KH_2PO_4$ (analytical grade) | 1.07 g |
| $K_2HPO_4 3H_2O$ (analytical grade) | 3.03 g |
| Disodium edetate dihydrate (analytical grade) | 0.20 g |
| Urease, freeze-dried powder, 50 U/mg (from Miles Laboratories Inc., code No. 36-615) | 0.40 g |
| DHD (as obtained above) | 13.00 ml |
| Methanol (analytical grade) | 25.00 ml |

Distilled water, to 100 ml of solution.

These impregnated sheets were thereafter dried and then cut in squares of 5 mm x 5 mm to form devices. The devices were then backed with double-faced adhesive tape and fixed thereby to plastic handles.

The test papers thus obtained were dipped in human serum or plasma and, after 12 minutes at room temperature, the yellow color obtained was visually compared with a suitable color scale: normal values of urea nitrogen did not give significant color response, while values higher than normal did. The same test papers were dipped human urine and, after 2 minutes at room temperature, the yellow color obtained was visually compared with a suitable color scale. Normal values of urea nitrogen did not give significant color response, while values higher than normal did. Neither plasma proteins nor substances present in urine other than urea interfered.

## Example IV

Assay of Urea Nitrogen in Body Fluid with Acetylacetone and Acetaldehyde

Reagent preparations used in the assay were as follows:

Reagent 1: the same as in Example 1

Reagent 2: the same as in Example 1

Reagent 3: 13.6 ml acetylacetone + 3.7 ml acetaldehyde (both analytical grade reagents) made up to 100 ml with distilled water.

A working solution was prepared as follows:

Reagent 1 + 2: the same as in Example 1

The determination was performed according to the kinetic two-point method. The scheme of the volumes used in the assay was the same as in Example 1. The reaction was carried out at 37°C and the absorbance at 390 nm read with an automated analyzer (1st reading after 30 seconds, 2nd reading after 10 minutes from the start). The calculation was the same as in Example 1. The method was linear up to at least 120 mg/dl urea nitrogen in the sample.

Recovery tests carried out adding known amounts of urea to a pool of human plasmas gave an average percent recovery of 98.8 in the range of linearity. Assays of

human sera or plasmas carried out in comparison with the urease/Berthelot method, gave a correlation coefficient r = 0.992 in the range of linearity.

Although the invention has been described with particularly, numerous changes in the details may be resorted to without departing from the scope of the invention.

CLAIMS:

1. Test means for the determination of urea in a sample which test means comprises urease, and an indicator reagent composition comprising acetylacetone and a $C_1$-$C_4$ alkyl aldehyde.

2. The test means of claim 1 wherein the $C_1$-$C_4$ alkyl aldehyde is formaldehyde.

3. The test means of claim 1 wherein the $C_1$-$C_4$ alkyl aldehyde is acetaldehyde.

4. Test means for the determination of urea in a sample which test means comprises urease, and an indicator reagent composition comprising 3,5-diacetyl-2,6-heptane-dione.

5. A test device for the determination of urea in a sample, which device comprises a carrier incorporated with a test means of any of claims 1 to 4.

6. A process for determination of urea in a sample which comprises contacting said sample with a test means of any of claims 1 to 4 and observing any resultant color formed.

7. A test kit for determining urea in a sample comprising, in packaged combination, a container of urease, and a container of an indicator reagent composition comprising acetylacetone and $C_1$-$C_4$ alkyl aldehyde.

8. The test kit of claim 7 wherein said $C_1$-$C_4$ alkyl aldehyde is formaldehyde.

9. The test kit of claim 7 wherein said $C_1$-$C_4$ alkyl aldehyde is acetaldehyde.

10. A test kit for the determination of urea in a sample comprising, in packaged combination, a container of urease and a container of 3,5-diacetyl-2,6-heptanedione.

0031051

European Patent Office

# EUROPEAN SEARCH REPORT

Application number
EP 80 10 7452

## DOCUMENTS CONSIDERED TO BE RELEVANT

| Category | Citation of document with indication, where appropriate, of relevant passages | Relevant to claim | CLASSIFICATION OF THE APPLICATION (Int. Cl.³) |
|---|---|---|---|
| X | RESEARCH DISCLOSURE, no. 169, May 1978, pages 48-52, no. 16954 Vant Hants, G.B. "Method, composition and element for the detection of nitrogen-containing compounds" * Page 48, column 2; page 49, column1; page 50; page 51, column 2 * -- | 1-10 | C 12 Q 1/58 |
| | US - A - 4 125 377 (E.M. GINDLER) * Abstract; column 6, claims 1, 5 * -- | 1-4,6-10,1-6 | TECHNICAL FIELDS SEARCHED (Int. Cl.³) C 12 Q 1/00 1/58 G 01 N 33/52 33/62 |
| | CHEMICAL ABSTRACTS, vol. 78, no. 25, 25th June 1973, page 147, no. 156196q Columbus, Ohio, U.S.A. W. GOEDICKE et al.: "Fluorometric determination of uric acid by use of the uricase-peroxidase system and 3,5-diacetyl-1,4-dihydroluti-dine as secondary substrate" & CLIN. CHIM. ACTA 1972, 44(2), 159-163 * Abstract * -- | 4,10 | |
| | CHEMICAL ABSTRACTS, vol. 82, no. 7, 17th February 1975, page 193, no. 40400e Columbus, Ohio, U.S.A. P. ROESCHLAU et al.: "Enzymic de-termination of total cholesterol in serum" & Z. KLIN. CHEM. KLIN. BIOCHEM. 1974, 12(9), 403-407 * Abstract * -- ./. | 1-3,6-9 | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 18-03-1981 | WALLINDER |

0031051

European Patent Office

# EUROPEAN SEARCH REPORT

Application number
EP 80 10 7452
-2-

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| D | CHEMICAL ABSTRACTS, vol. 48, no. 2, 25th January 1954, column 503i Columbus, Ohio, U.S.A. T. NASH: "Colorimetric estimation of formaldehyde by means of the Hantzsch reaction" & BIOCHEM.J. (London) 55, 416-421 1953 * Abstract * -- | 1-3,6-9 | |
| | CHEMICAL ABSTRACTS, vol. 66, no. 19, 8th May 1967, page 7762, no. 82993a Columbus, Ohio, U.S.A. F. DUNSBACH: "The determination of triglycerides in serum with the Hantzch reaction" & Z. KLIN. CHEM. 4(5), 262-264 1966 * Abstract * -- | 1-3,6-9 | TECHNICAL FIELDS SEARCHED (Int. Cl.3) |
| P,X | US - A - 4 194 063 (D.S. FRANK) * Abstract; column 19, claims 1,2; columns 21,22; claims 24-27 * ---- | 1-10 | |

EPO Form 1503.2 06.78